# EUROPEAN PATENT SPECIFICATION

(11) **EP 2 716 309 B1**
(45) Date of publication and mention of the grant of the patent: **08.03.2017**
(21) Application number: 12793202.8
(22) Date of filing: 31.05.2012
(51) Int. Cl.: A61M 1/14, C02F 1/44, A61M 1/16

(54) **BLOOD PURIFICATION SYSTEM**
BLUTREINIGUNGSSYSTEM
SYSTÈME DE PURIFICATION DE SANG

(30) Priority: 01.06.2011 JP 2011123392
(43) Date of publication of application: 09.04.2014
(73) Proprietor: Nikkiso Company Limited, Tokyo 150-6022 (JP)
(72) Inventor: KUCHIKI, Masaru, Makinohara-shi Shizuoka 421-0496 (JP); SUZUKI, Hiroaki, Makinohara-shi Shizuoka 421-0496 (JP); OKABE, Harutoshi, Makinohara-shi Shizuoka 421-0496 (JP)
(74) Representative: von Hirschhausen, Helge
(86) International application number: PCT/JP2012/064130
(87) International publication number: WO 2012/165566

(56) References cited:
- JP-A- 2008 023 324
- JP-A- 2008 220 784
- JP-B- 52 045 438
- JP-B2- 3 996 449
- JP-B2- 4 536 143
- JP-Y2- 6 009 668
- US-A- 4 596 549
- US-A1- 2010 181 235

## Description

### Technical Field

The present invention relates to a blood purification system which includes multiple blood purification means to which a blood purifier is attached in order to perform a blood purification treatment on a patient and supplying means capable of supplying a dialysate or an undiluted dialysate to each of the blood purification means.

### Background Art

In general, a blood purification system is configured such that a dialysate supplying device is installed in a machine room in a medical field such as a hospital, a dialysis monitoring device is installed in a separately arranged dialysis room (treatment room), and the dialysate supplying device and the dialysis monitoring device are connected to each other by a pipe. Whereas the dialysate supplying device produces a dialysate having a predetermined concentration by using supplied clean water, a plurality of dialysis monitoring devices are installed corresponding to the number of blood purifiers (dialyzers) to perform a dialysis treatment on a patient, introduce the dialysate produced by the dialysate supplying device through the pipe, and supply the dialysate to the blood purifiers.

That is, a configuration is made such that the dialysate is distributed and fed from the dialysate supplying device installed in the machine room to the plurality of dialysis monitoring devices installed in the dialysis room, and that the dialysate is supplied to the dialyzers in each dialysis monitoring device. Whereas a blood purification system which distributes the dialysate produced in the machine room as described above to each dialysis monitoring device is generally referred to as a "central system for a dialysis treatment", a device which can produce the dialysate for each of the blood purifiers (that is, each dialysis treatment patient) is generally referred to as a "personal dialysis device".

In the related art, a technique has been suggested in which the dialysate supplying device and each of the dialysis monitoring devices are electrically connected to each other and during a dialysis treatment process, a pipe cleaning process or a pipe disinfecting process, an electrical signal (process signal) is transmitted from the dialysate supplying device (supplying means) to each of the dialysis monitoring devices (blood purification means) (for example, refer to JP 200416412 A). In this manner, each of the dialysis monitoring devices, when receiving a predetermined electrical signal (process signal), performs an operation (driving of a pump or opening and closing of an electromagnetic valve) corresponding to the signal. A further example for such a blood dialyzing apparatus is disclosed in the document US 2010/181235 A1.

### Summary of Invention

### Technical Problem

However, in the above-described blood purification system in the related art, the electrical signal is transmitted from the dialysate supplying device (supplying means) to each of the dialysis monitoring devices (blood purification means) to perform a uniform operation. Thus, there has been a problem in that it is difficult to cause the dialysis monitoring devices to perform an individual operation depending on various situations. Similarly, such a problem also arises when the blood purification means is adapted to the personal dialysis device.

The present invention is made in view of such circumstances, and aims to provide a blood purification system that can cause blood purification means to perform an individual operation depending on various situations.

### Solution to Problem

According to the invention described in Claim 1, there is provided a blood purification system including multiple blood purification means to which a blood purifier is attached in order to perform a blood purification treatment on a patient; and supplying means capable of supplying a dialysate, an undiluted dialysate, clean water or an antiseptic solution to each of the blood purification means, wherein predetermined information can be transmitted from the supplying means to specified blood purification means out of the multiple blood purification means, and the specified blood purification means can be caused to perform an individual operation.

Further, depending on situations of the supplying means, the specified blood purification means is caused to be operated or stopped, or a drive amount of an actuator provided in the specified blood purification means is decreased, wherein the situations of the supplying means are selected from the group consisting of suppliable amounts of dialysate and undiluted dialysate or electric power.

Further, under a condition that the dialysate to be supplied from the supplying means or the undiluted dialysate to be supplied to the supplying means is determined to be insufficient, a use amount of the dialysate in the specified blood purification means is decreased and/or under a condition that electric power to be supplied to multiple blood purification means is determined to be insufficient, a use amount of the electric power in the specified blood purification means is decreased.

According to the invention described in Claim 2, in the blood purification system described in Claim 1, the supplying means is adapted to have a dialysate supplying device that can produce a dialysate having predetermined concentration by using clean water and an undiluted dialysate, and the blood purification means is adapted to have a monitoring device that supplies the dialysate supplied from the dialysate supplying device to the blood purifier.

According to the invention described in Claim 3, in the blood purification system described in Claim 1, the supplying means is adapted to have a water treatment device which can produce clean water, and the blood purification means is adapted to have a personal dialysis device that produces a dialysate having predetermined concentration by using the clean water supplied from the water treatment device and supplies the dialysate to the blood purifier.

According to the invention described in Claim 4, in the blood purification system described in Claim 1, the supplying means is adapted to have a water treatment device that can produce clean water and a dissolving device that can produce an undiluted dialysate having predetermined concentration by using the clean water produced in the water treatment device, and the blood purification means is adapted to have a personal dialysis device that produces a dialysate having predetermined concentration by using the undiluted dialysate and the clean water which are respectively supplied from the dissolving device and the water treatment device and supplies the dialysate to the blood purifier.

According to the invention described in Claim 5, in the blood purification system described in any one of Claims 1 to 4, the specified blood purification means is adapted to have predetermined number of blood purification means which is divided into a group out of multiple blood purification means.

According to the invention described in Claim 6, in the blood purification system described in Claim 5, the disinfecting is sequentially performed for each group by supplying the antiseptic solution from the supplying means to the specified blood purification means which is divided into the group.

According to the invention described in Claim 7, in the blood purification system described in any one of Claims 1 to 6, the specified blood purification means can be divided into a group for every one frequency of each blood purification treatment, and can be set to have disinfecting time according to a use frequency of the group.

According to the invention described in Claim 8, in the blood purification system described in
any one of Claims 1 to 7, the supplying means and the blood purification means can bilaterally communicate with each other.

### Advantageous Effects of Invention

According to the invention described in Claim 1, the predetermined information can be transmitted from the supplying means to the specified blood purification means out of multiple blood purification means, and the specified blood purification means can be caused to perform the individual operation. Therefore, depending on the various situations, the blood purification means can be caused to perform the individual operation.

Further, depending on situations of the supplying means, the specified blood purification means is caused to be operated or stopped, or the drive amount of the actuator provided in the specified blood purification means is decreased. Therefore, it is possible to cause the blood purification means to perform a more appropriate action depending on the various situations of the supplying means.

Further, under the condition that the dialysate to be supplied from the supplying means or the undiluted dialysate to be supplied to the supplying means is determined to be insufficient, the use amount of the dialysate in the specified blood purification means is decreased. Therefore, it is possible to cause the blood purification means to perform an individual action according to the dialysate or the undiluted dialysate.

Further, under the condition that the electric power to be supplied to multiple blood purification means is determined to be insufficient, the use amount of the electric power in the specified blood purification means is decreased. Therefore, it is possible to cause the blood purification means to perform an individual action according to the electric power supply.

According to the invention described in Claim 2, the supplying means is adapted to have a dialysate supplying device that can produce a dialysate having predetermined concentration by using clean water and an undiluted dialysate, and the blood purification means is adapted to have a monitoring device that supplies the dialysate supplied from the dialysate supplying device to the blood purifier. Therefore, the blood purification system can be applied to a central system.

According to the invention described in Claim 3, the supplying means is adapted to have the water treatment device which can produce clean water, and the blood purification means is adapted to have the personal dialysis device that produces the dialysate having predetermined concentration by using the clean water supplied from the water treatment device and supplies the dialysate to the blood purifier. Therefore, the blood purification system can be applied to a blood purification system having the personal dialysis device.

According to the invention described in Claim 4, the supplying means is adapted to have the water treatment device that can produce clean water and the dissolving device that can produce the undiluted dialysate having predetermined concentration by using the clean water produced in the water treatment device, and the blood purification means is adapted to have the personal dialysis device that produces the dialysate having predetermined concentration by using the undiluted dialysate and the clean water which are respectively supplied from the dissolving device and the water treatment device and supplies the dialysate to the blood purifier. Therefore, the blood purification system can be applied to a blood purification system having the personal dialysis device.

According to the invention described in Claim 5, the specified blood purification means is adapted to have the predetermined number of blood purification means which are divided into a group out of multiple blood purification means. Therefore, it is possible to more easily cause the blood purification means to perform an individual operation depending on the various situations.

According to the invention described in Claim 6, the disinfecting is sequentially performed for each group by supplying the antiseptic solution from the supplying means to the specified blood purification means which is divided into the group. Therefore, more efficient disinfecting can be performed.

According to the invention described in Claim 7, the specified blood purification means can be divided into a group for every one frequency of each blood purification treatment, and can be set to have disinfecting time according to a use frequency of the group. Therefore, much more efficient disinfecting can be performed according to the use frequency.

According to the invention described in Claim 8, the supplying means and the blood purification means can bilaterally communicate with each other. Therefore, various items of the information can be communicated between the supplying means and the blood purification means. It is possible to perform a more appropriate and safe blood purification treatment.

### Brief Description of Drawings

[Fig. 1] Fig. 1 is an overall schematic view illustrating a blood purification system according to an embodiment of the present invention.
[Fig. 2] Fig. 2 is a schematic view illustrating a configuration of a monitoring device in the blood purification system.
[Fig. 3] Fig. 3 is a flowchart illustrating control content (control when dialysate or undiluted dialysate is insufficient) of a dialysate supplying device in the blood purification system.
[Fig. 4] Fig. 4 is a flowchart illustrating control content (control when electric power is insufficient) of the dialysate supplying device in the blood purification system.
[Fig. 5] Fig. 5 is a schematic view illustrating divided groups in the blood purification system.
[Fig. 6] Fig. 6 is a flowchart illustrating control content (control of sequential disinfecting) of the dialysate supplying device in the blood purification system.
[Fig. 7] Fig. 7 is a flowchart illustrating control content (control of disinfecting according to a frequency of treatment) of the dialysate supplying device in the blood purification system.
[Fig. 8] Fig. 8 is an overall schematic view illustrating a blood purification system according to another embodiment of the present invention.
[Fig. 9] Fig. 9 is an overall schematic view illustrating a blood purification system according to still another embodiment of the present invention.

### Description of Embodiments

Hereinafter, an embodiment of the present invention will be described in detail with reference to the drawings.

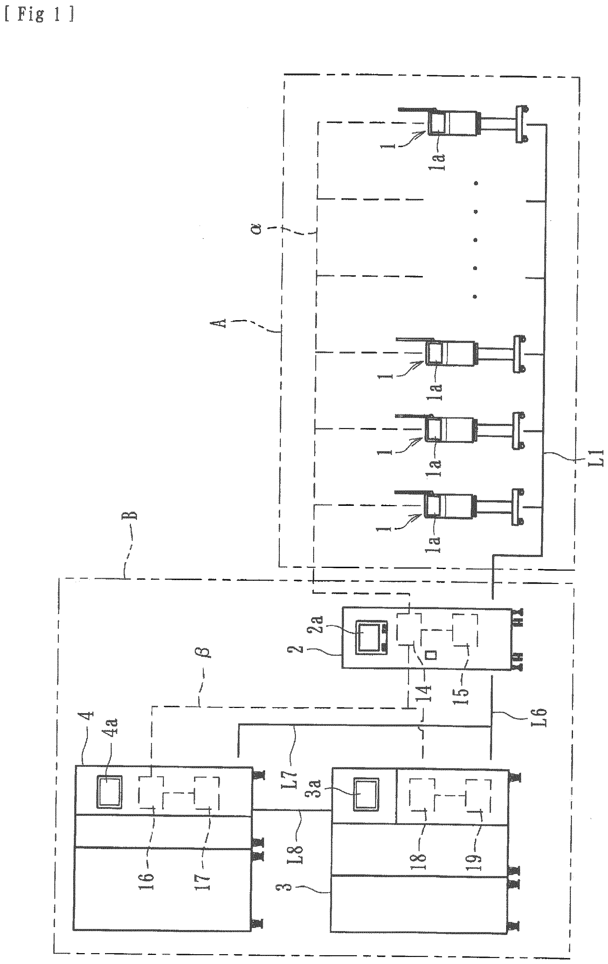

A blood purification system according to the present embodiment produces a dialysate having predetermined concentration using an undiluted dialysate and supplies the dialysate to a plurality of dialysis monitoring devices. As illustrated in Fig. 1, the blood purification system is mainly configured to include a plurality of monitoring devices 1 installed in a dialysis room A (treatment room) in a medical field such as a hospital; a dialysate supplying device 2 installed in a machine room B which is a place separated from the dialysis room A in the medical field; a dissolving device 3; and a water treatment device 4.

The monitoring device 1 (blood purification means) has a dialyzer 5 (blood purifier) attached thereto in order to perform a blood purification treatment (hemodialysis treatment) on a patient, and supplies the dialysate supplied from the dialysate supplying device 2 to the dialyzer 5. A plurality of monitoring devices 1 is installed at the dialysis room A. In the monitoring devices 1, touch panels 1a are arranged which can perform an instruction and a predetermined display of the hemodialysis treatment or other control content (cleaning or disinfecting).

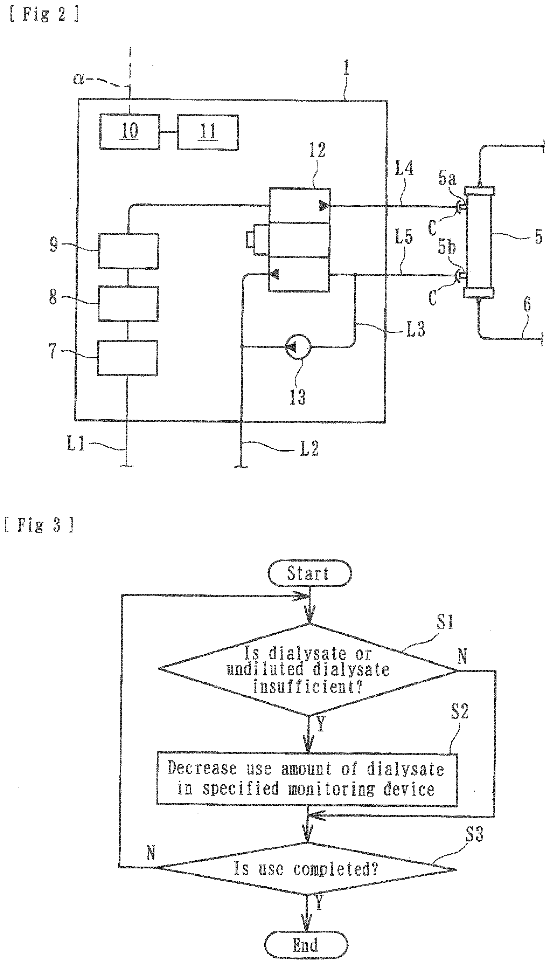

More specifically, as illustrated in Fig. 2, a pipe L1 extending from the dialysate supplying device 2 is drawn into each of the plurality of monitoring devices 1 installed at the dialysis room A, and includes a pipe L2 which is connected to a liquid discharge device (not illustrated). A duplex pump 12 is arranged across the pipes L1 and L2. Out of the pipes L1 and L2, in the pipe L1 inside the monitoring device 1, a flow rate detection sensor 7 which detects a flow rate of a liquid flowing in the pipe L1, a liquid pressure detection sensor 8 which detects a liquid supply pressure of the liquid, and a conductivity detection sensor 9 which detects conductivity (concentration) of the liquid are arranged.

In addition, a dialysate introduction line L4 communicating with the pipe L1 and a dialysate discharge line L5 communicating with the pipe L2 are extended from the duplex pump 12. A tip of the dialysate introduction line L4 can be connected to a dialysate introduction port 5a of the dialyzer 5 via a coupler C, and a tip of the dialysate discharge line L5 can be connected to a dialysate discharge port 5b of the dialyzer 5 via the coupler C. In this manner, the dialyzer 5 depending on each patient is attached to each monitoring device 1, and a blood circuit 6 which extracorporeally circulates blood of the patient is connected to the dialyzer 5.

A pump room of the duplex pump 12 is divided into a liquid supply side pump room connected to the pipe L1 and a discharge side pump room connected to the pipe L2 by a single plunger (not illustrated). The plunger performs reciprocation in the pump room, thereby supplying the dialysate or a cleaning solution which is supplied to the liquid supply side pump room to the dialyzer 5. Then, the dialysate inside the dialyzer 5 is sucked into the discharge side pump room. Further, a pipe L3 which bypasses the duplex pump 12 and communicates with the pipe L2 and the dialysate discharge line L5 is formed inside the monitoring device 1, and a water removal pump 13 is arranged in the middle of the pipe L3. It is possible to perform water removal on the blood of a patient which flows inside the dialyzer 5 by driving the water removal pump 13.

Instead of the duplex pump 12, a so-called chamber type may be used. As sensors such as the flow rate detection sensor 7, the liquid pressure detection sensor 8 or the conductivity detection sensor 9, any sensor may be arranged, or other generic sensor may be added thereto. Further, in the present embodiment, the sensors are arranged in the pipe L1, but may be arranged in the other pipe (for example, the pipe L2). For example, the sensors such as the flow rate detection sensor 7, the liquid pressure detection sensor 8 or the conductivity detection sensor 9 may be arranged in any one of the pipe L1 and the pipe L2, or may be arranged in both pipes.

The water treatment device 4 includes a module (purifying filter) having an inherent filtration membrane therein, and obtains clean water (RO water) by purifying raw water. The water treatment device 4 is configured to be capable of supplying the clean water to the dissolving device 3 by being connected to the dissolving device 3 via a pipe L8, and to be capable of supplying the clean water to the dialysate supplying device 2 by being connected to the dialysate supplying device 2 via pipes L6 and L7. The clean water obtained by the water treatment device 4 is used when the dialysate supplying device 2 produces the dialysate, or is used as the cleaning water for cleaning the pipes of the dialysate supplying device 2 or the monitoring devices 1. The configuration may also be made such that the water treatment device 4 is connected to a personal dialysis device (not illustrated) or a dissolving device which dissolve a powdered dialysis drug and supply the clean water thereto.

In addition, the water treatment device 4 includes a touch panel 4a which can perform an instruction and a predetermined display of control content relating to the water treatment, an interface unit 16 which is electrically connected to a LAN cable □, and a control unit 17. Then, if the interface unit 16 receives predetermined information from the dialysate supplying device 2, a predetermined operation (producing the clean water) is performed by the control unit 17 based on the information.

For example, a predetermined amount of powder drug for dialysis is dispensed to the dissolving device 3 which mixes the powder drug for dialysis with the clean water supplied from the water treatment device 4 and produces the undiluted dialysate having predetermined concentration. The dissolving device 3 includes a touch panel 3a which can perform an instruction and a predetermined display of control content relating to the producing of the undiluted dialysate, an interface unit 18 which is electrically connected to a LAN cable □ (to be described later), and a control unit 19. Then, if the interface unit 18 receives predetermined information from the dialysate supplying device 2, a predetermined operation (producing the undiluted dialysate) is performed by the control unit 19 based on the information. The dissolving device 3 is connected to the dialysate supplying device 2 via the pipe L6, and is configured to be capable of supplying the produced undiluted dialysate to the dialysate supplying device 2.

The dialysate supplying device 2, the dissolving device 3 and the water treatment device 4 are connected to each other on a local area network (LAN), thereby enabling bilateral information communication. The LAN is not limited to use of the LAN cable □ (wired LAN) as in the present embodiment, but may be any one which enables the bilateral information communication in a wireless manner (wireless LAN). Instead of the LAN, a system may be used which can transmit an electrical signal (process signal) unilaterally from the dialysate supplying device 2 to the dissolving device 3 and the water treatment device 4.

The dialysate supplying device 2 (supplying means) can produce the dialysate having predetermined concentration by using the clean water obtained from the water treatment device 4 and the undiluted dialysate produced by the dissolving device 3, and can supply the produced dialysate to each of the monitoring devices 1 (blood purification means). That is, the dialysate supplying device 2 is connected to each of the plurality of monitoring devices 1 via the pipe L1, and is configured to be capable of supplying a desired liquid such as the dialysate, the cleaning water and the antiseptic solution to each of the monitoring devices 1 via the pipe L1. The dialysate supplying device 2 includes a touch panel 2a which can perform an instruction and a predetermined display of control content relating to the supplying of the dialysate, the cleaning or the disinfecting.

Here, in the blood purification system according to the present embodiment, the dialysate supplying device 2 (supplying means) and each of the monitoring devices 1 (purification device) are connected to each other on the local area network (LAN), thereby enabling the bilateral information communication. In this configuration of the blood purification system, the predetermined information can be transmitted from the dialysate supplying device 2 to the specified monitoring device 1 out of the plurality of monitoring devices 1, and the specified monitoring device 1 can be caused to perform the individual operation.

That is, LAN connection is made via the LAN cable □ between the dialysate supplying device 2 and each of the monitoring devices 1. Whereas the dialysate supplying device 2 includes the interface unit 14 and the control unit 15 in order to communicate with the monitoring devices 1 via the LAN cable □, each of the monitoring devices 1 includes the interface unit 10 and the control unit 11 (refer to Fig. 2) in order to communicate with the dialysate supplying device 2 via the LAN cable □. The LAN is not limited to use of the LAN cable □ (wired LAN) as in the present embodiment, but a connection may be used which enables the bilateral information communication wirelessly (wireless LAN).

Further, in the dialysate supplying device 2, if the interface unit 14 receives the predetermined information from the monitoring devices 1, the predetermined operation (producing the dialysate) is performed by the control unit 15 based on the information, and the predetermined information can be transmitted to the monitoring devices 1 via the interface unit 14. In addition, in the monitoring devices 1, if the interface unit 10 receives the predetermined information from the dialysate supplying device 2, the predetermined operation (priming before treatment, hemodialysis treatment, re-transfusion, cleaning and disinfecting) is performed by the control unit 11 based on the information, and the predetermined information can be transmitted to the dialysate supplying device 2 via the interface unit 10.

Here, the predetermined information to be transmitted from the dialysate supplying device 2 to the specified monitoring device 1 out of the plurality of monitoring devices 1 is adapted to include information which can recognize situations different from a normal state (for example, suppliable dialysate and undiluted dialysate or insufficient electric power). In particular, the present invention is configured such that depending on situations (suppliable amounts of dialysate and undiluted dialysate or electric power) of the dialysate supplying device 2, the specified monitoring device 1 is caused to be operated or stopped, or the drive amount of the actuator (duplex pump 12 or water removal pump 13) provided in the specified monitoring device 1 is decreased, thereby selectively decreasing the flow rate of the liquid circulating in the pipe L1 or L2. In this manner, it is possible to perform a more appropriate action depending on the various situations of the dialysate supplying device 2 by operating or stopping the specified monitoring device 1, or by decreasing the drive amount of the actuator provided in the specified monitoring device 1, depending on the situations of the dialysate supplying device 2.

For example, under a condition that the dialysate supplied from the dialysate supplying device 2 or the undiluted dialysate supplied to the dialysate supplying device 2 is determined to be insufficient, by way of the communication among the dissolving device 3, the water treatment device 4 and each of the monitoring devices 1, the drive amount of the duplex pump 12 in the specified monitoring device 1 can be decreased to decrease the use amount of the dialysate. In this case, under the condition that the dialysate supplied from the dialysate supplying device 2 or the undiluted dialysate supplied to the dialysate supplying device 2 is determined to be insufficient, the use amount of the dialysate in the specified monitoring device 1 is decreased. Therefore, it is possible to perform an individual action according to the dialysate or the undiluted dialysate.

Hereby, under the condition that the dialysate supplied from the dialysate supplying device 2 or the undiluted dialysate supplied to the dialysate supplying device 2 is determined to be insufficient, the use amount of the dialysate in the specified monitoring device 1 is configured to be decreased. The control content in the dialysate supplying device 2 in this case will be described with reference to the flowchart in Fig. 3. By way of the communication among the dissolving device 3, the water treatment device 4 and each of the monitoring devices 1, the dialysate to be used is compared with the dialysate or the undiluted dialysate, each of which has been produced or can be produced. Then, it is determined whether or not the dialysate or the undiluted dialysate is insufficient (S1). If it is determined that the dialysate or the undiluted dialysate is insufficient, the step proceeds to S2. The predetermined information (drive control information of the duplex pump 12) is transmitted to the specified monitoring device 1 (in this case, the monitoring device 1 performing the dialysis treatment on a patient who can decrease the dialysate to be supplied), and the drive amount of the duplex pump 12 of the monitoring device 1 for the patient who can decrease the use of the dialysate is decreased. This leads to the decreased use amount of the dialysate of all the monitoring devices 1. In S1, when it is determined that the dialysate or the undiluted dialysate is sufficient, a process in S3 is performed by skipping S2.

Thereafter, it is determined whether or not the use of the dialysate (hemodialysis treatment) is completed (S3). If it is determined that the use of the dialysate is completed, a series of controls is completed. In contrast, if it is determined that the use of the dialysate is not completed, the step returns to S1 and it is determined again that the dialysate or the undiluted dialysate is insufficient. In the present embodiment, if it is determined that the dialysate or the undiluted dialysate is insufficient, the drive amount of the duplex pump 12 of the specified monitoring device 1 is decreased. However, as long as the use amount of the dialysate can be decreased, other configurations (in a case where a valve is provided which can control an individual liquid supply amount for each of the monitoring devices 1, the use amount of the dialysate is configured to be decreased by the control of the valve) may be adopted.

Further, for example, when power supply is switched to a private power generation due to a power failure in medical facilities, under a condition that the electric power to be supplied to the monitoring devices 1 is determined to be insufficient, it is possible to decrease the use amount of the electric power in the specified monitoring device 1. In this case, under a condition that the electric power to be supplied to the plurality of monitoring devices 1 is determined to be insufficient, the electric power for the dialysate in the specified monitoring device 1 is decreased. Therefore, it is possible to perform an individual action according to the power supply.

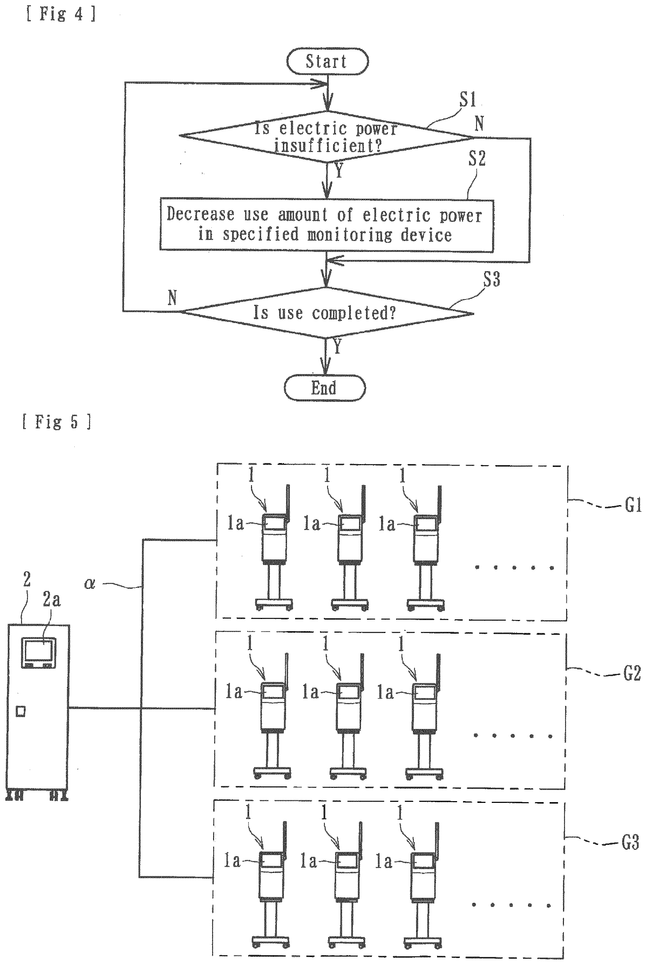

Hereby, under a condition that the electric power to be supplied to the monitoring devices 1 is determined to be insufficient, the use amount of the dialysate in the specified monitoring device 1 is configured to be decreased. The control content in the dialysate supplying device 2 in this case will be described with reference to the flowchart in Fig. 4. When a decrease in the power supply to the blood purification system is detected, it is determined whether or not the electric power which can be supplied to the monitoring devices 1 is insufficient (S1). If it is determined that the electric power is insufficient, the step proceeds to S2. Then, the predetermined information (drive control information of the duplex pump 12) is transmitted to the specified monitoring device 1 (in this case, the monitoring device 1 which performs a dialysis treatment on a patient who can decrease the dialysate to be supplied), and the drive amount of the duplex pump 12 of the monitoring device 1 in the patient who can decrease the use of the dialysate is decreased. This leads to a decrease in the use amount of the electric power for all the monitoring devices 1. In S1, when it is determined that the electric power is sufficient, a process in S3 is performed by skipping S2.

Thereafter, it is determined whether or not the use of the dialysate (hemodialysis treatment) is completed (S3). If it is determined that the use of the dialysate is completed, a series of controls is completed. In contrast, if it is determined that the use of the dialysate is not completed, the step returns to S1 and it is determined again that the electric power is insufficient. In the present embodiment, if it is determined that the electric power is insufficient, the drive amount of the duplex pump 12 in the specified monitoring device 1 is decreased. However, as long as the electric power to be used can be decreased, other configurations (configuration to decrease the power supply to other configuring elements such as a heating device in each of the monitoring devices 1) may be adopted.

For example, in S2, the predetermined information (operation control information of a heater) is transmitted to the specified monitoring device 1 (in this case, the monitoring device 1 which can decrease the electric power to be supplied to the heater such as the heating device), thereby enabling a decrease in the use amount of the electric power. That is, in general, when cleaning and disinfecting processes are switched to a substitution process (process where the liquid inside the pipe is substituted with the dialysate instead of the cleaning water or the antiseptic solution), or when the power supply is restored after the power failure, the heaters in all the monitoring devices 1 are simultaneously operated. Accordingly, power consumption is excessively increased for the time being. However, it is possible to suppress a peak of the power consumption by decreasing the operation of the heater in the specified monitoring device 1.

Furthermore, as illustrated in Fig. 5, the specified monitoring device 1 may be adapted to have a predetermined number of monitoring devices 1 which are divided into groups G1 to G3 out of the plurality of monitoring devices 1. That is, the predetermined information can be transmitted from the dialysate supplying device 2 to the specified monitoring device 1 belonging to predetermined groups (G1 to G3) out of the plurality of monitoring devices 1, and the specified monitoring device 1 of each group can be caused to perform an individual operation. In this manner, if the specified monitoring device 1 is adapted to have the predetermined number of monitoring devices 1 which are divided into groups out of the plurality of monitoring devices 1, it is possible to more easily cause the monitoring devices 1 to perform the individual operation depending on various situations.

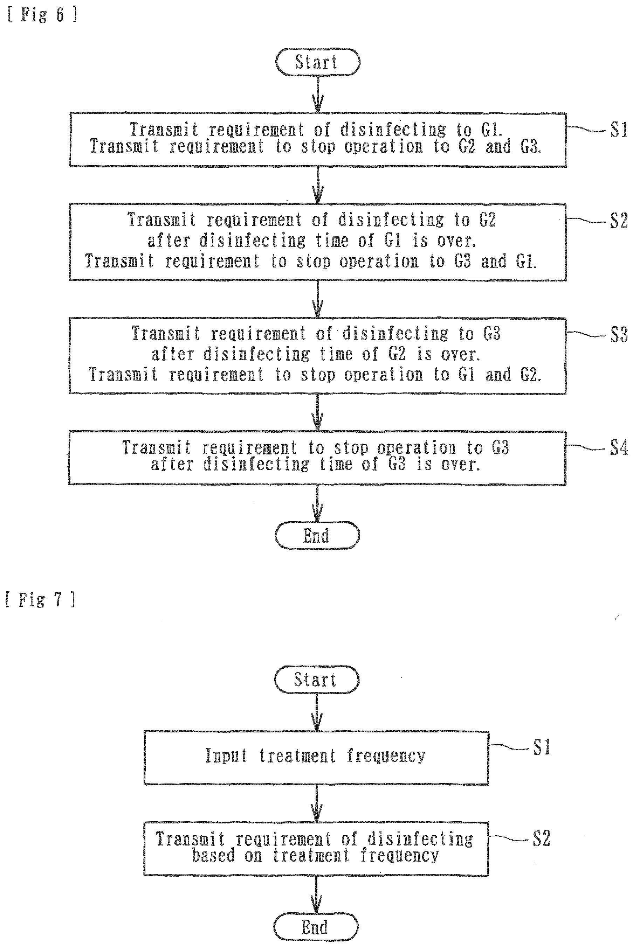

For example, as illustrated in Fig. 5, the respective monitoring devices 1 are divided into the groups G1 to G3, and the disinfecting can be sequentially performed on each group by supplying the antiseptic solution from the dialysate supplying device 2 to the specified monitoring device 1 which is divided into a group. In this case, the disinfecting is sequentially performed on each group by supplying the antiseptic solution from the dialysate supplying device 2 to the specified monitoring device 1 which is divided into the group. Therefore, it is possible to more efficiently perform the disinfecting. Hereinafter, the control content in the dialysate supplying device 2 in this case will be described with reference to the flowchart in Fig. 6.

First, a requirement is transmitted from the dialysate supplying device 2 to the specified monitoring device 1 belonging to G1, and the disinfecting is performed on the monitoring device 1 of G1, and a requirement to stop an operation is transmitted to the monitoring devices 1 belonging to the other groups (G2 and G3) (S1). Thereafter, under a condition that disinfecting time is over for the monitoring device 1 belonging to G1, the disinfecting requirement is transmitted from the dialysate supplying device 2 to the specified monitoring device 1 belonging to G2, the disinfecting is performed on the monitoring device 1 belonging to G2, and the requirement to stop the operation is transmitted to the monitoring device 1 belonging to the other groups (G1 and G3) (S2).

Subsequently, under a condition that the disinfecting time is over for the monitoring device 1 belonging to G2, the disinfecting requirement is transmitted from the dialysate supplying device 2 to the specified monitoring device 1 belonging to G3, the disinfecting is performed on the monitoring device 1 belonging to G3, and the requirement to stop the operation is transmitted to the monitoring devices 1 belonging to the other groups (G1 and G2) (S3). Then, under a condition that the disinfecting time is over for the monitoring device 1 belonging to G3, the requirement to stop the operation is transmitted from the dialysate supplying device 2 to the monitoring device 1 belonging to G3 (S4).

In this manner, a series of controls during the disinfecting is completed. By completing the above-described controls and sequentially performing the disinfecting on the monitoring device 1 of each group, it is possible to efficiently perform the disinfecting. In particular, when the inside of the pipe is sterilized by using hot antiseptic solution or hot water as the antiseptic solution, the disinfecting is performed by concentrating on the monitoring device 1 which is divided into each group. Accordingly, it is possible to suppress a decrease in a temperature of the antiseptic solution, and it is possible to more efficiently perform the disinfecting.

For example, as illustrated in Fig. 5, the respective monitoring devices 1 can be divided into the groups G1 to G3 according to the frequency of the hemodialysis treatments, and the disinfecting time can be set according to the use frequency of the group. In this case, the specified monitoring device 1 can be divided into a group for each frequency of the respective hemodialysis treatments (blood purification treatment), and the disinfecting time can be set according to the use frequency of the group. Accordingly, it is possible to more efficiently perform the disinfecting according to the use frequency. Hereinafter, the control content in the dialysate supplying device 2 in this case will be described with reference to the flowchart in Fig. 7.

For example, a treatment frequency of the monitoring device 1 is input to the dialysate supplying device 2 in advance by operating the touch panel 2a (S1). Then, for example, the antiseptic solution is supplied to the monitoring device 1 belonging to a group which is frequently used for the treatment until a long time (for example, 60 minutes) elapses so as to perform the disinfecting. The antiseptic solution is supplied to the monitoring device 1 belonging to a group which is less frequently used for the treatment until a short time (for example, 30 minutes) elapses so as to perform the disinfecting (S2).

In this manner, a series of controls during the disinfecting is completed. By completing the above-described controls and sequentially performing the disinfecting on the monitoring device 1 of each group according to the treatment frequency, it is possible to efficiently perform the disinfecting. In the above-described embodiment, the disinfecting time is set according to the treatment frequency. However, instead of the treatment frequency, the disinfecting time may be set according to pipe capacity in the monitoring device 1.

According to the above-described embodiment, the predetermined information can be transmitted from the dialysate supplying device 2 to the specified monitoring device 1 out of the plurality of monitoring devices 1, and the specified monitoring device 1 can be caused to perform the individual operation. Therefore, the monitoring devices 1 can be caused to perform the individual operation depending on various situations such as power supply conditions and undiluted dialysate conditions. In addition, it is preferable to configure a control means 11 such that in the monitoring device 1 to which the predetermined information is transmitted, operations are individually determined so as to further improve safety. Furthermore, according to the present embodiment, the supplying means is adapted to have the dialysate supplying device 2 which can produce the dialysate having the predetermined concentration by using the clean water and the undiluted dialysate, and the blood purification means is adapted to have the monitoring devices 1 which supplies the dialysate supplied from the dialysate supplying device 2 to the dialyzer 5 (blood purifier). Therefore, the blood purification system can be applied to a central system. In addition, since the monitoring devices 1 and the dialysate supplying device 2 can bilaterally communicate with each other, various items of the information can be communicated between the monitoring devices 1 and dialysate supplying device 2. It is possible to perform a more appropriate and safe blood purification treatment.

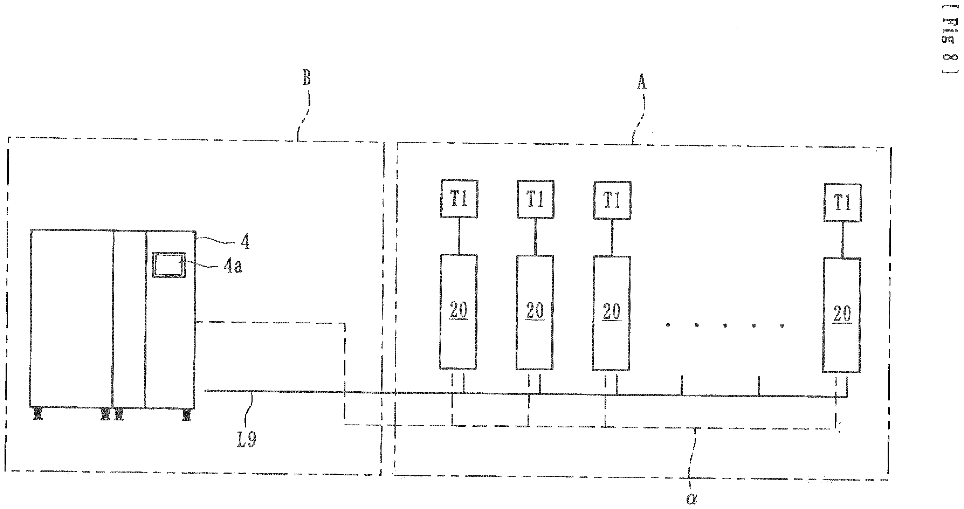

Hitherto, the blood purification system according to the present embodiment has been described, but the present invention is not limited thereto. For example, instead of those adapted to have the central system as described above, as illustrated in Fig. 8, the blood purification system may be applied to a system where the supplying means is adapted to have the water treatment device 4 which can produce the clean water, and adapted to have personal dialysis devices 20 which produce the dialysate having the predetermined concentration by using the clean water supplied from the water treatment device 4 and which supply the dialysate to the dialyzer 5 (blood purifier). In this case, the personal dialysis devices 20 include tanks T1 respectively containing the undiluted dialysate or the antiseptic solution, and are connected to the water treatment device 4 by a pipe L9. The personal dialysis devices 20 can produce the dialysate having the predetermined concentration by using the clean water supplied from the water treatment device 4 and the undiluted dialysate inside the tanks T1, or can produce the diluted antiseptic solution.

That is, in the embodiment illustrated in Fig. 1, the supplying means is configured to have the dialysate supplying device 2 (including the dissolving device 3 and the water treatment device 4) which can supply the dialysate to each of the monitoring devices 1. However, the blood purification system may be configured such that the blood purification means has the dialyzer 5 (blood purifier) and the personal dialysis device 20 which can produce the dialysate from the undiluted dialysate inside the tanks T1.

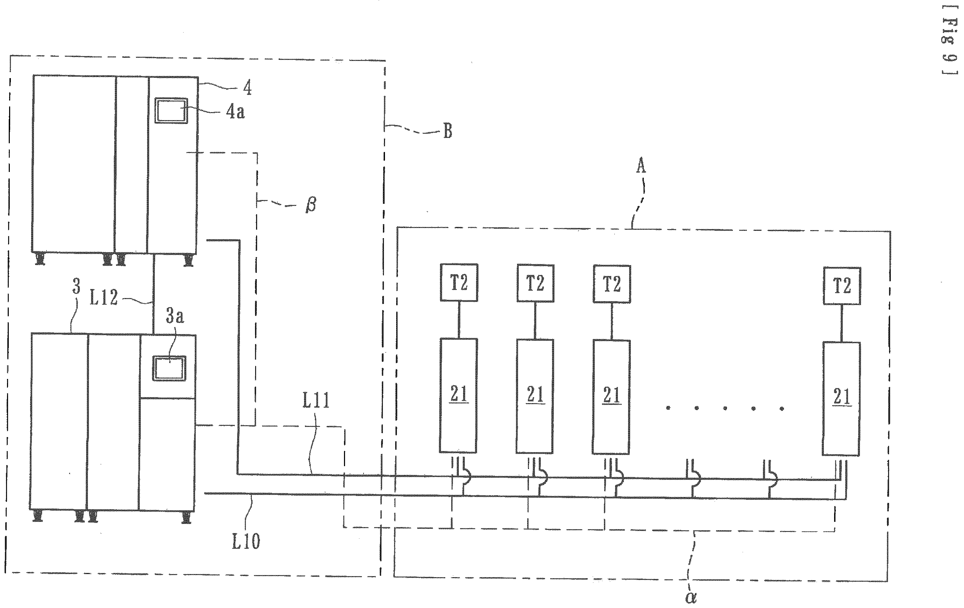

Furthermore, for example, instead of those adapted to have the central system, as illustrated in Fig. 9, the blood purification system may be applied to a system where the supplying means is adapted to have the water treatment device 4 which can produce the cleaning water and the dissolving device 3 which can produce the undiluted dialysate having the predetermined concentration by using the clean water produced by the water treatment device 4, and the blood purification means is adapted to have personal dialysis devices 21 which produce the dialysate having the predetermined concentration by using the undiluted dialysate and the clean water which are respectively supplied from the dissolving device 3 and the water treatment device 4, and which supply the dialysate to the dialyzer 5 (blood purifier). In this case, the personal dialysis devices 21 include tanks T2 respectively containing the antiseptic solution, and are connected to the dissolving device 3 and the water treatment device 4 by pipes L10 and L11. The personal dialysis devices 21 can produce the dialysate having the predetermined concentration by using the undiluted dialysate and the clean water which are respectively supplied from the dissolving device 3 and the water treatment device 4, or can produce the diluted antiseptic solution.

That is, in the embodiment illustrated in Fig. 1, the supplying means is configured to have the dialysate supplying device 2 (including the dissolving device 3 and the water treatment device 4) which can supply the dialysate to each of the monitoring devices 1. However, the blood purification system may be configured such that the blood purification means is adapted to have the dialyzer 5 (blood purifier) and the personal dialysis devices 21 which can produce the dialysate by using the undiluted dialysate and the clean water which are supplied from the supplying means side.

In addition, in the present embodiment, the LAN connection is made to enable bilateral communication between the dialysate supplying device 2 and the monitoring devices 1. However, it is sufficient if the predetermined information can be transmitted from the dialysate supplying device 2 to the specified monitoring device 1 out of the plurality of monitoring devices 1 (blood purification means) and the specified monitoring device 1 can be caused to perform the individual operation. The predetermined information may be unilaterally transmitted from the dialysate supplying device 2 to the monitoring device 1. The present embodiment is applied to a system to perform the hemodialysis treatment, but may be applied to a blood purification system to perform a different blood purification treatment.

### Industrial Applicability

As long as a blood purification system is configured such that predetermined information can be transmitted from supplying means to a specified blood purification means out of multiple blood purification means and the specified blood purification means can be caused to perform an individual operation, the blood purification system can be applied to those having other added functions.

### Reference Signs List

- 1: monitoring device (blood purification means)
- 2: dialysate supplying device (supplying means)
- 3: dissolving device
- 4: water treatment device
- 5: dialyzer (blood purifier)
- 6: blood circuit
- 7: flow rate detection sensor
- 8: liquid pressure detection sensor
- 9: conductivity detection sensor
- 10: interface unit
- 11: control unit
- 12: duplex pump
- 13: water removal pump
- 14: interface unit
- 15: control unit
- 16: interface unit
- 17: control unit
- 18: interface unit
- 19: control unit

## Claims

1. A blood purification system comprising: multiple blood purification means (1) to which a blood purifier (5) is attached in order to perform a blood purification treatment on a patient; and supplying means (2) capable of supplying a dialysate, an undiluted dialysate, clean water or an antiseptic solution to each of the blood purification means (1), wherein predetermined information may be transmitted from the supplying means (2) to a specified blood purification means (1) out of multiple blood purification means (1), and the specified blood purification means may be caused to perform an individual operation,
wherein, depending on situations of the supplying means (2), the specified blood purification means (1) is caused to be operated or stopped, or a drive amount of an actuator provided in the specified blood purification means (1) is decreased, wherein the situations of the supplying means are selected from the group consisting of suppliable amounts of dialysate and undiluted dialysate or electric power,
**characterized in that**
when under a condition that the dialysate to be supplied from the supplying means (2) or the undiluted dialysate to be supplied to the supplying means (2) is determined to be insufficient, a use amount of the dialysate in the specified blood purification means (1) is decreased, and/or
when under a condition that electric power to be supplied to multiple blood purification means (1) is determined to be insufficient, a use amount of the electric power in the specified blood purification means (1) is decreased.

2. The blood purification system according to claim 1, wherein the supplying means (2) is adapted to have a dialysate supplying device (2) that may produce a dialysate having predetermined concentration by using clean water and an undiluted dialysate, and wherein the blood purification means (1) is adapted to have a monitoring device (1) that supplies the dialysate supplied from the dialysate supplying device (2) to the blood purifier (5).

3. The blood purification system according to claim 1, wherein the supplying means (2) is adapted to have a water treatment device (4) which may produce clean water, and wherein the blood purification means (1) is adapted to have a personal dialysis device that produces a dialysate having predetermined concentration by using the clean water supplied from the water treatment device (4) and supplies the dialysate to the blood purifier (5).

4. The blood purification system according to claim 1, wherein the supplying means (2) is adapted to have a water treatment device (4) that may produce clean water and a dissolving device (3) that may produce an undiluted dialysate having predetermined concentration by using the clean water produced in the water treatment device (4), and wherein the blood purification means (1) is adapted to have a personal dialysis device that produces a dialysate having predetermined concentration by using the undiluted dialysate and the clean water which are respectively supplied from the dissolving device (3) and the water treatment device (4) and supplies the dialysate to the blood purifier.

5. The blood purification system according to any one of claims 1 to 4, wherein the specified blood purification means (1) is adapted to have predetermined number of blood purification means (1) which are divided into a group out of multiple blood purification means (1).

6. The blood purification system according to claim 5, wherein the disinfecting is sequentially performed for each group by supplying the antiseptic solution from the supplying means (2) to the specified blood purification means (1) which is divided into the group.

7. The blood purification system according to any one of claims 1 to 6, wherein the specified blood purification means (1) may be divided into a group depending on frequency of each blood purification treatment, and may be set to have disinfecting time according to a use frequency of the group.

8. The blood purification system according to any one of claims 1 to 7, wherein the supplying means (2) and the blood purification means (1) may bilaterally communicate with each other.

## Patentansprüche

1. Blutreinigungssystem, aufweisend: mehrere Blutreinigungseinrichtungen (1), an denen ein Blutreiniger (5) angebracht ist, um eine Blutreinigungsbehandlung bei einem Patienten durchzuführen; und eine Zuführeinrichtung (2), die fähig ist, ein Dialysat, ein unverdünntes Dialysat, Reinwasser oder eine antiseptische Lösung zu jeder von den Blutreinigungseinrichtungen (1) zuzuführen, wobei vorbestimmte Information von der Zuführeinrichtung (2) zu einer bezeichneten Blutreinigungseinrichtung (1) aus den mehreren Blutreinigungseinrichtungen (1) übertragen werden kann, und die bezeichnete Blutreinigungseinrichtung veranlasst werden kann, eine individuelle Operation durchzuführen, wobei, abhängig von Gegebenheiten der Zuführeinrichtung (2), veranlasst wird, dass die bezeichnete Blutreinigungseinrichtung in Betrieb gesetzt oder gestoppt wird, oder ein Antriebsausmaß eines Stellantriebs, der in der bezeichneten Blutreinigungseinrichtung (1) vorgesehen ist, vermindert wird, wobei die Gegebenheiten der Zuführeinrichtung aus der Gruppe gewählt sind, die aus zuführbaren Mengen an Dialysat und unverdünntem Dialysat oder elektrischem Strom besteht,
**dadurch gekennzeichnet, dass**
wenn, unter einer Bedingung, dass bestimmt wird, dass das von der Zuführeinrichtung (2) zuzuführende Dialysat oder das der Zuführeinrichtung (2) zuzuführende unverdünnte Dialysat nicht ausreichend ist, eine Verwendungsmenge an Dialysat in der bezeichneten Blutreinigungseinrichtung (1) vermindert wird und/oder
wenn, unter der Bedingung, dass bestimmt wird, dass ein elektrischer Strom, der mehreren Blutreinigungseinrichtungen (1) zugeführt werden soll, nicht ausreichend ist, eine Verwendungsmenge an elektrischem Strom in der bezeichneten Blutreinigungseinrichtung (1) vermindert wird.

2. Blutreinigungssystem nach Anspruch 1, wobei die Zuführeinrichtung (2) ausgebildet ist, eine Dialysatzuführvorrichtung (2) zu haben, die ein Dialysat von vorbestimmter Konzentration erzeugen kann, und zwar unter Verwendung von Reinwasser und unverdünntem Dialysat, und wobei die Blutreinigungseinrichtung (1) ausgebildet ist, eine Überwachungsvorrichtung (1) aufzuweisen, die das von der Dialysatzuführvorrichtung (2) zugeführte Dialysat dem Blutreiniger (5) zuführt.

3. Blutreinigungssystem nach Anspruch 1, wobei die Zuführeinrichtung (2) ausgebildet ist, eine Wasserbehandlungsvorrichtung (4) aufzuweisen, die Reinwasser erzeugen kann, und wobei die Blutreinigungseinrichtung (1) ausgebildet ist, eine persönliche Dialysevorrichtung aufzuweisen, die ein Dialysat von vorbestimmter Konzentration erzeugt, und zwar unter Verwendung des von der Wasserbehandlungsvorrichtung (4) zugeführten Reinwassers, und das Dialysat dem Blutreiniger (5) zuführt.

4. Blutreinigungssystem nach Anspruch 1, wobei die Zuführeinrichtung (2) ausgebildet ist, eine Wasserbehandlungsvorrichtung (4), die Reinwasser erzeugen kann, und eine Lösungsvorrichtung (3) aufzuweisen, die ein unverdünntes Dialysat von vorbestimmter Konzentration erzeugen kann, und zwar unter Verwendung des in der Wasserbehandlungsvorrichtung (4) erzeugten Reinwassers, und wobei die Blutreinigungseinrichtung (1) ausgebildet ist, eine persönliche Dialysevorrichtung aufzuweisen, die ein Dialysat von vorbestimmter Konzentration erzeugt, und zwar unter Verwendung des unverdünnten Dialysats und des Reinwassers, die von der Lösungsvorrichtung (3) bzw. der Wasserbehandlungsvorrichtung (4) zugeführt werden, und das Dialysat dem Blutreiniger zuführt.

5. Blutreinigungssystem nach einem der Ansprüche 1 bis 4, wobei die bezeichnete Blutreinigungseinrichtung (1) ausgebildet ist, um eine vorbestimmte Anzahl von Blutreinigungseinrichtungen (1) aufzuweisen, die aus mehreren Blutreinigungseinrichtungen (1) zu einer Gruppe aufgeteilt wurde.

6. Blutreinigungssystem nach Anspruch 5, wobei das Desinfizieren in sequentieller Weise für jede Gruppe durchgeführt wird, und zwar dadurch, dass die antiseptische Lösung von der Zuführeinrichtung (2) zu der bezeichneten Blutreinigungseinrichtung (1) zugeführt wird, die zu der Gruppe aufgeteilt wurde.

7. Blutreinigungssystem nach einem der Ansprüche 1 bis 6, wobei die bezeichnete Blutreinigungseinrichtung (1) abhängig von der Häufigkeit einer jeweiligen Blutreinigungsbehandlung zu einer Gruppe aufgeteilt sein kann, und es festgelegt sein kann, dass sie eine Desinfektionszeit gemäß einer Nutzungshäufigkeit der Gruppe aufweist.

8. Blutreinigungssystem nach einem der Ansprüche 1 bis 7, wobei die Zuführeinrichtung (2) und die Blutreinigungseinrichtung (1) miteinander bilateral kommunizieren können.

## Revendications

1. Système de purification de sang comprenant : des moyens de purification de sang multiples (1) sur lesquels un purificateur de sang (5) est fixé afin de réaliser un traitement de purification de sang sur un patient ; et un moyen d'alimentation (2) pouvant fournir un dialysat, un dialysat non dilué, de l'eau pure ou une solution antiseptique à chacun des moyens de purification de sang (1), dans lequel une information prédéterminée peut être transmise du moyen d'alimentation (2) à un moyen de purification de sang (1) spécifique hors des moyens de purification de sang multiples (1) et le moyen de purification de sang spécifié peut être amené à réaliser une opération individuelle,
dans lequel, en fonction des situations du moyen d'alimentation (2), le moyen de purification de sang (1) spécifié est amené à être actionné ou arrêté ou une quantité d'entraînement d'un actionneur prévu dans le moyen de purification de sang (1) spécifié est réduite, dans lequel les situations du moyen d'alimentation sont sélectionnées dans le groupe comprenant des quantités pouvant être fournies de dialysat et de dialysat non dilué ou d'énergie électrique,
**caractérisé en ce que** :
lorsque, dans une condition dans laquelle on détermine que le dialysat à fournir au moyen d'alimentation (2) ou le dialysat non dilué à fournir au moyen d'alimentation (2) est insuffisant, une quantité d'utilisation du dialysat dans le moyen de purification de sang (1) spécifié est diminuée, et/ou
lorsque, dans une condition dans laquelle on détermine que l'énergie électrique à fournir aux moyens de purification de sang multiples (1) est insuffisante, une quantité d'utilisation d'énergie électrique dans le moyen de purification de sang (1) spécifié est augmentée.

2. Système de purification de sang selon la revendication 1, dans lequel le moyen d'alimentation (2) est adapté pour avoir un dispositif d'alimentation en dialysat (2) qui peut produire un dialysat ayant une concentration prédéterminée en utilisant de l'eau pure et un dialysat non dilué, et dans lequel le moyen de purification de sang (1) est adapté pour avoir un dispositif de surveillance (1) qui amène le dialysat alimenté à partir du dispositif d'alimentation en dialysat (2), au purificateur de sang (5).

3. Système de purification de sang selon la revendication 1, dans lequel le moyen d'alimentation (2) est adapté pour avoir un dispositif de traitement d'eau (4) qui peut produire de l'eau pure, et dans lequel le moyen de purification de sang (1) est adapté pour avoir un dispositif de dialyse personnel qui produit un dialysat ayant une concentration prédéterminée en utilisant l'eau pure alimentée à partir du dispositif de traitement d'eau (4) et amène le dialysat au purificateur de sang (5).

4. Système de purification de sang selon la revendication 1, dans lequel le moyen d'alimentation (2) est adapté pour avoir un dispositif de traitement d'eau (4) qui peut produire de l'eau pure et un dispositif de dissolution (3) qui peut produire un dialysat non dilué ayant une concentration prédéterminée en utilisant l'eau pure produite dans le dispositif de traitement d'eau (4) et dans lequel le moyen de purification de sang (1) est adapté pour avoir un dispositif de dialyse personnel qui produit un dialysat ayant une concentration prédéterminée en utilisant le dialysat non dilué et l'eau pure qui sont respectivement alimentés à partir du dispositif de dissolution (3) et le dispositif de traitement d'eau (4) et amène le dialysat au purificateur de sang.

5. Système de purification de sang selon l'une quelconque des revendications 1 à 4, dans lequel le moyen de purification de sang (1) spécifié est adapté pour avoir un nombre prédéterminé de moyens de purification de sang (1) qui sont divisés en un groupe hors des moyens de purification de sang multiples (1).

6. Système de purification de sang selon la revendication 5, dans lequel la désinfection est réalisée de manière séquentielle pour chaque groupe en fournissant la solution antiseptique à partir du moyen d'alimentation (2) au moyen de purification de sang (1) spécifié qui est divisé dans le groupe.

7. Système de purification de sang selon l'une quelconque des revendications 1 à 6, dans lequel le moyen de purification de sang (1) spécifié peut être divisé en un groupe en fonction de la fréquence de chaque traitement de purification de sang et peut être réglé pour avoir le temps de désinfection selon une fréquence d'utilisation du groupe.

8. Système de purification de sang selon l'une quelconque des revendications 1 à 7, dans lequel le moyen d'alimentation (2) et le moyen de purification de sang (1) peuvent communiquer de manière bilatérale entre eux.
